(19) Europäisches Patentamt
European Patent Office
Office européen des brevets

(11) **EP 4 635 523 A1**

(12) **EUROPEAN PATENT APPLICATION**
published in accordance with Art. 153(4) EPC

(43) Date of publication:
22.10.2025 Bulletin 2025/43

(21) Application number: 23903528.0

(22) Date of filing: 13.12.2023

(51) International Patent Classification (IPC):
*A61L 27/06* (2006.01)   *B21C 1/00* (2006.01)
*C22C 14/00* (2006.01)   *C22F 1/00* (2006.01)
*C22F 1/18* (2006.01)

(52) Cooperative Patent Classification (CPC):
A61L 27/06; B21C 1/00; C22C 14/00; C22F 1/00;
C22F 1/18

(86) International application number:
PCT/JP2023/044585

(87) International publication number:
WO 2024/128244 (20.06.2024 Gazette 2024/25)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 14.12.2022 JP 2022199634

(71) Applicant: Maruemu Works Co., Ltd.
Osaka-shi, Osaka 5420086 (JP)

(72) Inventors:
• YAMANAKA, Shigeru
Daitou-shi, Osaka 574-0015 (JP)
• SHINOHARA, Ryo
Daitou-shi, Osaka 574-0015 (JP)
• FUKUDA, Kenji
Daitou-shi, Osaka 574-0015 (JP)

(74) Representative: Meissner Bolte Partnerschaft
mbB
Patentanwälte Rechtsanwälte
Postfach 86 06 24
81633 München (DE)

(54) **BASE MATERIAL FOR SCREW, SCREW, AND METHOD FOR PRODUCING SAME**

(57)    The present invention provides a pure titanium screw or a base material for a pure titanium screw having sufficient strength comparable to that of titanium alloys. The present invention provides a substantially cylindrical pure titanium screw base material or screw, wherein the maximum value of the specific strength in the orientation of the (1 0 -1 0) plane in the axial direction of the substantially cylindrical shape is 3 or more, and the hardness of a center part and the hardness of an outer peripheral part in a substantially circular cross section orthogonal to the axial direction are approximately the same.

Fig. 1

**Description**

Technical Field

The present invention relates to a base material for a screw or a screw and a producing method thereof, particularly a base material for a medical screw or a medical screw and a producing method thereof, more particularly a base material for a medical anchor screw or a medical anchor screw and a producing method thereof, most particularly, a base material for an orthodontic anchor screw or an orthodontic anchor screw and a producing method thereof.

Background Art

A medical screw made of titanium has become important in medical implants. Most of them are made of alloy titanium (Ti64). Titanium alloys (for example, Ti-6Al-4V) have had problems with allergies, especially due to vanadium, since vanadium is one of the alloying elements.

In industrial products, for example, during the step of cleaning a substrate in which special acids and solutions are used, that require cleanliness and corrosion resistance, and during the step of producing a film in which the film is subjected to vacuum or special gas environments in the semiconductor industry, a screw made of pure titanium has been used, since titanium alloys such as Ti-6Al-4V are inferior to pure titanium in corrosion resistance, and also since elution of aluminum and vanadium, which are additive elements of titanium alloys, occurs and thus titanium alloys may cause contamination with impurities. On the other hand, since pure titanium has low strength, it is necessary to make up for the lack of strength by increasing the size of the screw or increasing the number of screws, and thus there is a need to improve the strength of pure titanium itself.

As a method for improving the strength of pure titanium, for example, Patent Documents 1 and 2 disclose that titanium or a titanium alloy as an implant was swaged to improve mechanical properties. Further, Patent Document 1 discloses appropriate processing conditions and processing ratio. However, Patent Documents 1 and 2 show the advantages of general working strengthening common to metal plastic working. They also show that the processing ratio is preferably 20 to 80%, and thus that if it is greater than 80%, it becomes brittle and cracks occur during processing.

More, Patent Document 2 discloses the characteristics of the processing mode of swaging, but it is only qualitative and cannot be said to be sufficient from the point of view of reliability.

Patent Document 3 discloses a technique for improving the mechanical properties of titanium by methods such as warm rolling, extrusion, and die forging. The method involves using the cyclic shear deformation method (ECAP), which is one of the crystal refinement and strengthening methods of titanium, to create a material by controlling the temperature while heating from the surroundings, and then performs rolling, which is the main secondary processing, to enhance the effect. The features of Patent Document 3 reside in grain refinement and improved crystal isotropy.

Further, Patent Document 4 discloses that after refining titanium by a Multi-Directional Forging process (MDF), it is subjected to rolling and rod processing, wherein the processing temperature at that time is 70 °C or less, to realize increased strength.

More, Non-Patent Document 1 discloses that for pure titanium Grades 1 to 4, starting from materials that have been refined by structural changes due to heat treatment such as quenching, they are further strengthened by processing.

In addition, Patent Document 5 uses a processing method called hydrostatic extrusion. However, as shown in Non-Patent Document 2, in the hydrostatic extrusion, there are parts (periphery region) where the grain flow line during processing is not parallel to the central axis of the material, and the characteristics vary depending on the part of the material, making it difficult to obtain uniform characteristics. In other words, while the central axis is subject to axial elongation deformation, the surface area, which receives a tangential force from the die, is dominated by shear deformation, so the characteristics vary depending on the part of the material. This is also the case with drawing, which receives a large tangential force from the die during processing. In addition, in the hydrostatic extrusion processing method, for the same reason, the maximum value of the specific strength of the orientation changes between the center and the periphery, and when averaged over the cross section, the maximum value of the specific strength does not exceed 3, making it impossible to obtain the required characteristics.

More, Patent Document 6 discloses that high-strength, filamentous crystal pure titanium can be produced only by providing the following steps of:

(1) adjusting the grain orientation of pure titanium by using one or two equal-diameter angular extrusions, to obtain a titanium bar;
(2) performing rotary forging on the titanium bar obtained in the step (1) multiple times and then cutting the bar, to obtain a titanium plate having a rectangular cross section;
(3) subjecting the titanium plate to repeat of an annealing and multiple times of controlled rolling process; and
(4) annealing the titanium plate.

Although Patent Document 6 can obtain pure titanium with high toughness, it requires a complicated process. Because of the complicated process, the grain flow of the obtained titanium material also has a complicated flow. Further, since the process is complicated, such as crystals being precipitated and/or grown in the pure titanium by annealing (heat treatment), it is difficult to obtain a material with stable properties in the same material as a whole or between lots.

Further, in processing methods in which shear deformation occurs on the surface and the cross section is reduced, the hardness varies between the center and the periphery of the cross section, making it difficult to obtain a uniform hardness in the material. In order to obtain a material with a uniform hardness, a complex processing method was required. For example, Non-Patent Document 3 can be cited as an example of drawing processing (see especially the description on page 94 of Non-Patent Document 3).

Prior Art Document

Non-Patent Document

Non-Patent Document 1: Hiroaki Matsumoto, Heisei 26 Research Results Report (Public Interest Incorporated Foundation, The Kyoto Technoscience Center), "New type of ultrafine-grained microstructural formation technique of Pure-Ti and its development for practical application"
Non-Patent Document 2: Kenzo KATO, KINZOKU SOSEI KAKOUGAKU (Metal Plastic Processing), Maruzen Co., Ltd., 1979, p. 208-212.
Non-Patent Document 3: Anton Pomp: "Steel Wire", Wire industry Ltd, (1954).

Patent Document

Patent Document 1: JP-A-H7-124242.
Patent Document 2: JP-A-H9-135852.
Patent Document 3: JP Patent No. 5536789.
Patent Document 4: JP Patent No. 6737686.
Patent Document 5: JP-A-2016-506387.
Patent Document 6: JP-A-2021-508764.

Summary of Invention

Problems to be solved by the Invention

Although pure titanium is the metal with the lowest allergy risk, it lacks the tensile strength and torsional rupture strength required for medical screws compared to titanium alloys. Since minimal invasiveness is required, it is not preferable to increase the strength by increasing the size, and thus the strength of the material itself is required.

Conventional technology tried to use the work hardening and fine strengthening characteristics of pure titanium to improve mechanical properties, but the technology was insufficient. Recently, after making a special grain refining strengthened material through bulk ultrafine grained (UFG) processing, the material is subjected to rolling processing, to make a cylindrical shape (bar material or wire material) for medical screws. Therefore, the process is complicated, and there are restrictions on the shape and volume of the material subjected to huge strain, and thus a low-cost method having excellence in productivity has been needed.

On the other hand, commercial pure titanium (CP titanium), which is commercially available as a bar or wire, is easily available. However, since crystal grains of CP titanium are as large as several tens of microns and thus CP titanium does not have sufficient strength, and since there are some variations in internal structure thereof, it has been necessary to have quality control to achieve stable production and high reliability even when using CP titanium.

Accordingly, an object of the present invention is to provide a base material for a screw or a screw, particularly a base material for a medical screw or a medical screw, more particularly a base material for a medical anchor screw or a medical anchor screw, most particularly a base material for an orthodontic anchor screw or an orthodontic anchor screw, each of which is made of pure titanium, and has sufficient strength comparable to that of a titanium alloy.

Another object of the present invention is, in addition to or other than the above objects, to provide a method for producing a base material made of pure titanium for a screw, or a screw made of pure titanium, or the like.

Specifically, an object of the present invention is to provide an enable production from commercially available pure titanium bars or wires without going through a special process such as bulk ultrafine grained processing, and to provide a method capable of producing the above-described base material made of pure titanium for a screw, or the above-described screw made of pure titanium, or the like, by using a stable production method and a highly reliable management method.

Further, another object of the present invention is, in addition to or other than the above objects, to provide a base material made of pure titanium for a screw, or a screw made of pure titanium, each having a substantially cylindrical shape, each of which has torsional break torque and wear resistance, for example, required for a self-drilling screw, specifically the desired hardness, through the hardness of a central part and a peripheral part of a substantially circular cross section in a substantially circular cross section perpendicular to the axial direction of the base material or the screw with a substantially cylindrical shape being substantially same, and to provide a method for producing the base material or the screw.

More, another object of the present invention is, in addition to or other than the above objects, to provide a base material made of pure titanium for a screw, or a screw made of pure titanium, each of which has the wear resistance, specifically hardness, more specifically surface hardness and/or internal hardness, required for self-drilling, and which has strength against torsional torque, specifically torsional breaking strength, and to provide a method for producing the base material or the screw.

Further, another object of the present invention is, in addition to or other than the above objects, to provide a method for producing a base material made of pure titanium for a screw, or a screw made of pure titanium, with a relatively small number of steps, which can obtain a material having stable properties throughout the same material or between lots.

Means for Solving Problems

The present inventors have found the following inventions:

<1> A base material for a screw having a substantially cylindrical shape or a screw having a substantially cylindrical shape, wherein each of the base material and the screw is made of pure titanium, and each of the base material and the screw has 3 or more, preferably 4 or more, more preferably 5 or more of a maximum specific intensity of the orientation in the (1 0 -1 0) plane in the axial direction of the substantially cylindrical shape, and a hardness of a central part and a hardness of a peripheral part in a substantially circular cross section perpendicular to the axial direction are substantially same, the difference between the hardness of the central part and the peripheral part may preferably be within 15 HV, more preferably within 12 HV, most preferably within 10 HV. Furthermore, regarding the term "(1 0 -1 0) plane", the details will be mentioned later.

<2> In the above item <1>, when the total weight of the base material or the screw is normalized as 100 % by weight, an oxygen content in the base material or the screw may be 0.2 to 0.4 % by weight, preferably 0.25 to 0.38 % by weight, more preferably 0.28 to 0.37 % by weight, most preferably 0.29 to 0.36 % by weight.

<3> In the above item <1> or <2>, a crystallite size of the pure titanium may be 280 Å or less, preferably 270 Å or less, more preferably 260 Å or less.

<4> In any one of the above items <1> to <3>, each of the base material and the screw may have at least one, two, or three of the following properties i) to iii):

Property i): tensile strength of 800 MPa or more, preferably 860 MPa or more, more preferably 920 MPa or more;
Property ii): surface hardness of 200 HV or more, preferably 220 HV or more, more preferably 240 HV or more; and
Property iii): reduction of area of 45% or more, preferably 50% or more, more preferably 60% or more.

<5> In any one of the above items <1> to <4>, the pure titanium may be selected from the group consisting of pure titanium Grade 2, pure titanium Grade 3, pure titanium Grade 4, and pure titanium with crystal grains refined to 1 $\mu$m or less.

<6> In any one of the above items <1> to <5>, the pure titanium may be pure titanium Grade 4.

<7> In any one of the above items <1> to <6>, the base material or the screw may be a screw, particularly a self-drilling screw, and an aspect ratio of the titanium crystals in the central part in a plane parallel to the axial direction of the screw and the plane including the axis may be larger than an aspect ratio of the titanium crystals in the peripheral part, the aspect ratio of the central part may preferably be 1.10 to 1.50 times, more preferably 1.20 to 1.50 times, most preferably 1.30 to 1.50 times larger than the aspect ratio of the peripheral part.

<8> In any one of the above items <1> to <7>, the base material or the screw is a screw, particularly a self-drilling screw, and the base material or the screw may have a surface hardness and/or an internal hardness (specifically, the hardness of the central part and/or the hardness of the peripheral part) of 250 HV or more, preferably 270 HV or more, specifically 270 to 340 HV, more preferably 290 HV or more, specifically 290 to 320 HV.

<9> In any one of the above items <1> to <8>, the base material for the screw or the screw may be a base material for a medical anchor screw or a medical anchor screw.

<10> In any one of the above items <1> to <9>, the base material for the screw or the screw may be a base material for an orthodontic anchor screw or an orthodontic anchor screw.

<11> A method for producing a base material for a screw, comprising the steps of:

(I) preparing a pure titanium material having a substantially cylindrical shape and a cross-sectional area of A0; and
(II) swaging the pure titanium material, to obtain the base material for the screw, which has a substantially cylindrical shape having a cross-sectional area of A1 after swaging and a true strain represented by ln(A0/A1) of 2 or more, preferably 2.5 or more, more preferably 3.0 or more, wherein the maximum value of the specific intensity of the orientation of the (1 0 -1 0) plane in the axial direction of the substantially cylindrical shape is 3 or more, preferably 4 or more, and more preferably 5 or more, and a hardness of a central part and a peripheral part in a substantially circular cross section perpendicular to the axial direction are substantially same, and preferably the difference between the hardness of the central part and the peripheral part is 15 HV or less, more preferably 12 HV or less, and most preferably 10 HV or less.

<12> A method for producing a base material for a screw, consisting of:

(I) preparing a pure titanium material having a substantially cylindrical shape and a cross-sectional area of A0; and
(II) swaging the pure titanium material, to obtain the base material for the screw, which has a substantially cylindrical shape having a cross-sectional area of A1 after swaging and a true strain represented by ln(A0/A1) of 2 or more, preferably 2.5 or more, more preferably 3.0 or more, wherein the maximum value of the specific intensity of the orientation of the (1 0 -1 0) plane in the axial direction of the substantially cylindrical shape is 3 or more, preferably 4 or more, and more preferably 5 or more, and a hardness of a central part and a peripheral part in a substantially circular cross section perpendicular to the axial direction are substantially same, and preferably the difference between the hardness of the central part and the peripheral part is 15 HV or less, more preferably 12 HV or less, and most preferably 10 HV or less.

<13> A method for producing a screw, further comprising the step of (III) imparting a screw shape to the base material for the screw having the substantially cylindrical shape obtained in the above item <11> or <12>; to obtain the screw.

<14> In the step (III) of the above item <13>, the screw shape may be imparted by cutting the base material having the substantially cylindrical shape.

<15> In any one of the above items <11> to <14>, the method may further comprise the step of (IV) molding the base material for the screw at 250 °C or less, to form a screw head.

<16> In any one of the above items <11> to <15>, when the total weight of the base material or the screw is normalized as 100 % by weight, an oxygen content in the base material or the screw may be 0.2 to 0.4 % by weight, preferably 0.25 to 0.38 % by weight, more preferably 0.28 to 0.37 % by weight, most preferably 0.29 to 0.36 % by weight.

<17> In any one of the above items <11> to <16>, the crystallite size of pure titanium may be 280 Å or less, preferably 270 Å or less, more preferably 260 Å or less.

<18> In any one of the above items <11> to <17>, each of the base material for the screw and the screw may be made of pure titanium, and the crystallite size of pure titanium may be 280 Å or less, preferably 270 Å or less, more preferably 260 Å or less.

<19> In any one of the above items <11> to <18>, each of the base material for the screw and the screw may have at least one, two, or three of the following properties i) to iii):

Property i): tensile strength of 800 MPa or more, preferably 860 MPa or more, more preferably 920 MPa or more;
Property ii): surface hardness of 200 HV or more, preferably 220 HV or more, more preferably 240 HV or more; and
Property iii): reduction of area of 45% or more, preferably 50% or more, more preferably 60% or more.

<20> In any one of the above items <11> to <19>, the pure titanium may be selected from the group consisting of pure titanium Grade 2, pure titanium Grade 3, pure titanium Grade 4, and pure titanium with crystal grains refined to 1 $\mu$m or less.

<21> In any one of the above items <11> to <20>, the pure titanium may be pure titanium Grade 4.

<22> In any one of the above items <11> to <21>, the base material or the screw is a screw, particularly a self-drilling screw, and an aspect ratio of the titanium crystals in the central part in a plane parallel to the axial direction of the screw and the plane including the axis may be larger than an aspect ratio of the titanium crystals in the peripheral part, the aspect ratio of the central part may preferably be 1.10 to 1.50 times, more preferably 1.20 to 1.50 times, most preferably 1.30 to 1.50 times larger than the aspect ratio of the peripheral part.

<23> In any of the above items <11> to <22>, the base material or the screw is a screw, particularly a self-drilling screw, and the base material or the screw may have a surface hardness and/or an internal hardness (specifically, the hardness of the central part and/or the hardness of the peripheral part) of 250 HV or more, preferably 270 HV or more, specifically 270 to 340 HV, more preferably 290 HV or more, specifically 290 to 320 HV.

<24> In any one of the above items <11> to <23>, each of the base material for the screw and the screw may be a base material for a medical anchor screw or a medical anchor screw.

<25> In any one of the above items <11> to <24>, each of the screw base material and the screw may be a base material for an orthodontic anchor screw or an orthodontic anchor screw.

Effects of the Invention

The present invention can provide a base material for a screw or a screw, particularly a base material for a medical screw or a medical screw, more particularly a base material for a medical anchor screw or a medical anchor screw, most particularly a base material for an orthodontic anchor screw or an orthodontic anchor screw, each of which is made of pure titanium, and has sufficient strength comparable to that of a titanium alloy.

Further, other than or in addition to the above effect, the present invention can provide a method for producing a base material made of pure titanium for a screw, or a screw made of pure titanium, or the like.

Specifically, the present invention can provide an enable production from commercially available pure titanium bars or wires without going through a special process such as bulk ultrafine grained processing, and to provide a method capable of producing the above base material made of pure titanium for a screw, or a screw made of pure titanium, or the like, by using a stable production method and a highly reliable management method.

Further, in addition to or other than the above effects, the present invention can provide a base material made of pure titanium for a screw, or a screw made of pure titanium, each having a substantially cylindrical shape, each of which has torsional break torque and wear resistance, for example, required for a self-drilling screw, specifically the desired hardness, through the hardness of a central part and a peripheral part of a substantially circular cross section in a substantially circular cross section perpendicular to the axial direction of the base material or the screw with a substantially cylindrical shape being substantially same, and can provide a method for producing the base material or the screw.

More, in addition to or other than the above effects, the present invention can provide a base material made of pure titanium for a screw, or a screw made of pure titanium, each of which has the wear resistance, specifically hardness, more specifically surface hardness and/or internal hardness, required for self-drilling, and which has strength against torsional torque, specifically torsional breaking strength, and can provide a method for producing the base material or the screw.

Further, in addition to or other than the above effects, the present invention can provide a method for producing a base material made of pure titanium for a screw, or a screw made of pure titanium, with a relatively small number of steps, which can obtain a material having stable properties throughout the same material or between lots.

Brief Description of Drawings

Fig. 1 are figures, each of which shows the (1 0 -1 0) plane of the processed base materials having rod shape of the Examples obtained by preparing pure titanium Grade 2 (CP-T2), pure titanium Grade 4 (CP-T4), and FTi2 materials at true strains of 0, 2.0 and 3.7 as a pole figure by using analysis software DIFFRAC. TEXTURE MRDB V4.1 (manufactured by BRUKER).

Fig. 2 is a graph representing the crystallite size (Å) on the horizontal axis and the tensile strength (MPa) on the vertical axis for the processed base materials each having rod shape obtained in Examples.

Fig. 3 is a graph representing the crystallite size (Å) on the horizontal axis and the hardness (HV) on the vertical axis for the processed base materials each having rod shape obtained in Examples.

Fig. 4 is a graph representing crystallite size (Å) on the horizontal axis and reduction of area (%) on the vertical axis for the processed base materials each having rod shape obtained in Examples.

Fig. 5 is a graph representing the true strain ε on the horizontal axis and the crystallite size (Å) on the vertical axis for the processed base materials each having rod shape obtained in Examples.

Fig. 6 is a graph representing the true strain ε on the horizontal axis and the maximum specific intensity (orientation) on the vertical axis for the processed base materials each having rod shape obtained in Examples.

Fig. 7 is a graph representing the maximum specific intensity (orientation) on the horizontal axis and the tensile strength (MPa) on the vertical axis for the processed base materials each having rod shape obtained in Examples.

Fig. 8 is a graph representing the maximum specific intensity (orientation) on the horizontal axis and the hardness (HV) on the vertical axis for the processed base materials each having rod shape obtained in Examples.

Fig. 9 is a graph representing the maximum specific intensity (orientation) on the horizontal axis and the reduction of area (%) on the vertical axis for the processed base materials each having rod shape obtained in Examples.

Fig. 10 is a graph representing the true strain $\varepsilon$ on the horizontal axis and the reduction of area (%) on the vertical axis for the base material obtained using a material CP-T4.

Fig. 11 is a diagram showing the points at which the "internal hardness", i.e., the hardness of the central part and the hardness of the peripheral part, were measured in a substantially circular cross section perpendicular to the axial direction of a rod-shaped processed base material.

Fig. 12 is a photograph of a head processed on the base material obtained using a material CP-T4 processed with a true strain of 0 and a true strain of 2.65.

Fig. 13 is a diagram showing a schematic diagram of the measured surface and the locations thereof for the screw according to the present invention, as well as the results of the aspect ratio of the obtained titanium crystals.

Fig. 14 is a graph showing the oxygen contents on the horizontal axis and the hardness of the central part on the vertical axis for screws obtained using ii) CP-T4 as the material (oxygen contents: 0.28 % by weight and 0.34 % by weight); and screws obtained using FTi2 (oxygen contents: 0.12 % by weight and 0.14 % by weight).

Fig. 15 is a graph showing the oxygen contents on the horizontal axis and the torsional strength on the vertical axis for screws obtained using ii) CP-T4 as the material (oxygen contents: 0.28 % by weight and 0.34 % by weight); and screws obtained using FTi2 (oxygen contents: 0.12 % by weight and 0.14 % by weight).

Description of Embodiments

The invention described in the present application (hereinafter sometimes abbreviated as "the present invention") will be described hereinafter.

The present application provides a base material for a screw which is made of pure titanium and has a substantially cylindrical shape, a screw which is made of pure titanium and has substantially cylindrical shape, a method for producing the base material for the screw, and a method for producing the screw.

They will be described in order, hereinafter.

<A base material for a screw>

The present application provides a base material for a screw which is made of pure titanium and has a substantially cylindrical shape, wherein the base material has 3 or more, preferably 4 or more, more preferably 5 or more of a maximum specific intensity of the orientation in the (1 0 -1 0) plane in the axial direction of the substantially cylindrical shape. Further, the maximum specific intensity of the orientation may be 15 or less, preferably 12 or less, more preferably 10 or less.

Furthermore, the term "(1 0 -1 0)" used herein is usually represented by following (X). However, in the present specification, the term "(1 0 -1 0)" is used for convenience.

$$(1010) \qquad (X)$$

The "substantially cylindrical shape" includes not only a cylindrical shape but also a so-called truncated cone shape in which the side surface is inclined along the axial direction of the cylindrical shape.

One "made of pure titanium" is not limited to one that does not contain any impurities, but may be pure titanium Grade 1, pure titanium Grade 2, pure titanium Grade 3, or pure titanium Grade 4 according to JIS standard. Further, it may be pure titanium with crystal grains refined to 1 $\mu$m or less.

Furthermore, the material of "pure titanium" is preferably selected from the group consisting of pure titanium Grade 2, pure titanium Grade 3, pure titanium Grade 4, and pure titanium with crystal grains refined to 1 $\mu$m or less.

<Orientation in the (1 0 -1 0) plane in the axial direction of the substantially cylindrical shape>

The base material for the screw according to the present invention has 3 or more of a maximum specific intensity of the orientation in the (1 0 -1 0) plane in the axial direction of the substantially cylindrical shape. Further, the maximum specific intensity may be preferably 4 or more, more preferably 5 or more. More, the maximum specific intensity of the orientation may be 15 or less, preferably 12 or less, more preferably 10 or less.

The term "axial direction" has the same definition as the length direction of the substantially cylindrical shape.

Pure titanium is usually isotropic (or equiaxed crystal), but can be oriented by processing. The orientation imparts properties that cannot be obtained with an isotropic structure (or equiaxed crystal), to the screw base material. A specific crystal plane is preferentially aligned in the axial direction by a force applied perpendicularly to the outer peripheral surface of the raw material toward the center of the raw material for obtaining a base material for a screw having substantially cylindrical shape with a circular cross section. As a result, the strength in the axial direction is generally increased, and the tensile strength of the base material for the screw and the screw formed from the base material can be improved.

Conventionally, the orientation of pure titanium has been treated as a qualitative factor related to mechanical properties, rather than being treated quantitatively, although the orientation is a fundamental property along with crystallites. In order quantitatively to evaluate the orientation, X-ray diffraction (XRD) is used to quantitatively treat a specific crystal plane of pure titanium in the direction of interest as the maximum value of the ratio of intensity to average intensity, to find out treating the orientation quantitatively.

The present inventors found that, with respect to the characteristic crystal plane (1 0 -1 0) of pure titanium having a close-packed hexagonal crystal, a pole figure in the direction (axial direction) perpendicular to the cross section of a material having substantially cylindrical shape is created, and that the maximum specific intensity in the pole figure, which is the "maximum value of the specific intensity of the orientation in the (1 0 -1 0) plane in the axial direction of the substantially cylindrical shape", is obtained.

A pole figure shows how specific crystal planes of a material are distributed in a cross section of the material, and its intensity is generally indicated by a contour map or shading. The maximum specific intensity is the ratio of the intensity of the darkest part to the average. The higher the specific intensity is, the higher the orientation (also referred to as anisotropy) is. The value closer to 1 means the less orientation and the isotropic or random distribution.

The maximum value of specific intensity can be obtained by using X-ray diffraction (XRD), as described above. As a specific method of determination, D8 ADVANCE manufactured by BRUKER is used as an X-ray diffraction device, cobalt is used for the tube, and the output is set at a voltage of 35 kV and a current of 40 mA. A two-dimensional detector is used with a divergence slit diameter of 0.3 mm and a collimator diameter of 0.3 mm.

In creating the pole figure, the in-plane direction angle $\Phi$ of the sample is measured in 72 steps in 5 degree increments around 360 degrees, and the range of the tilt angle $\Psi$ is determined by measuring the starting point at 15 degrees and the ending point at 45 degrees. The obtained measurement data are analyzed using the analysis software DIFFRAC.TEXTURE MRDB V4.1 (manufactured by BRUKER), to create the pole figure of (1 0 -1 0) showing the characteristic behavior in the orientation of pure titanium. As a result, it is set so that the direction in which the crystal planes are most oriented can be understood by the color density. The maximum value of the relative intensities to the average intensity, which is obtained from the entire pole figure and is defined as 1, is defined as the maximum specific intensity. If the material is isotropic (no orientation) there will be less color shading, and orientation will produce darker areas at certain angles and higher relative intensities at those angles. The angle at which the intensity reaches the maximum value can also be found using a contour map.

According to the present invention, as described above, the "maximum value of the specific intensity in the orientation in the (1 0 -1 0) plane in the axial direction of the substantially cylindrical shape" may be 3 or more, preferably 4 or more, and more preferably 5 or more.

In the base material for screw or the screw according to the present invention, the hardness of the central part and the hardness of the peripheral part in a substantially circular cross section perpendicular to the axial direction may be substantially same, and the difference between the hardness of the central part and the peripheral part may be within 15 HV, more preferably 12 HV, most preferably 10 HV.

The characteristics can provide desired torsional break torque and hardness, and desired wear resistance, and thus, makes it possible to use the screw, particularly a self-drilling screw.

Furthermore, the hardness of the central part and the peripheral part in the substantially circular cross section perpendicular to the axial direction of the base material or the screw may be referred to as an "internal hardness" used herein.

On the other hand, the surface hardness or the hardness of the exterior part of the base material or the screw may sometimes be referred to as a "surface hardness" or "external hardness".

The "hardness" used herein refers to Vickers hardness for both "internal hardness", "surface hardness" and "external hardness".

The Vickers hardness can be determined by a conventional method, for example, in accordance with JIS Z 2244, using a Vickers hardness tester.

For example, among the "internal hardness", the hardness of the central part in the substantially circular cross section perpendicular to the axial direction of the base material or the screw can be determined by polishing the central part.

Regarding the "internal hardness", the hardness of the peripheral part in the substantially circular cross section perpendicular to the axial direction of the base material or the screw is determined at a position at a distance of 1/4 of the radius from the surface side toward the center.

The "surface hardness" or "external hardness" can be determined by measuring the hardness of the surface or the surface that is exposed to the outside in the same manner as described above. When the surface is curved, it is preferable to scrape the curved surface slightly to make it flat, and then press an indenter into the flat surface to measure the Vickers hardness.

The "surface hardness" or "external hardness" may be 200 HV or more, preferably 220 HV or more, more preferably 240 HV or more. When the screw according to the present invention is used as a self-drilling screw, the "surface hardness" or "external hardness" and/or the "internal hardness" (specifically, the hardness of the central portion and/or the hardness of the outer periphery) may be 250 HV or more, preferably 270 HV or more, specifically from 270 to 340 HV, more preferably 290 HV or more, specifically from 290 to 320 HV.

A self-drilling screw means a screw that is fixed by drilling a hole in an object into which the screw is to be embedded through a drill-shaped tip of the screw while forming a female thread with the subsequent screw. In contrast to a self-drilling screw, a self-tapping screw means a screw that is fixed by drilling a pilot hole, which should be a female thread, in an object into which the screw is to be embedded, followed by screwing the screw thereinto.

The base material for screw or the screw according to the present invention may have an oxygen content of 0.2 to 0.4 % by weight, preferably 0.25 to 0.38 % by weight, more preferably 0.28 to 0.37 % by weight, most preferably 0.29 to 0.36 % by weight, when the total weight of the base material or the screw is normalized as 100 % by weight.

The oxygen content having the above range and the base material for screw or the screw having the above properties can provide the desired wear resistance, specifically the desired hardness, more specifically the desired "surface hardness" or "external hardness", and/or the desired "internal hardness", as well as the desired strength against torsional torque, specifically the desired torsional breaking strength, and make it more suitable for use as a self-drilling screw.

The oxygen content in the base material or the screw according to the present invention can be determined, for example, by the inert gas fusion-infrared absorption method in accordance with JIS H1620-1995 (a method for determining an oxygen content in titanium and titanium alloys).

<Mechanical properties>

High strength and high toughness are required for a screw, especially a medical screw. The characteristics are also required for a base material for a screw.

Regarding the strength, the tensile strength in the axial direction of the screw, that is, the axial direction of the base material for the screw is important.

The tensile strength may be 800 MPa or higher, preferably 860 MPa or higher, more preferably 920 MPa or higher.

It may be preferably 820 MPa or more in order to make it nearly equivalent to alloy titanium (for example, Ti-6% Al-4%V). Depending on the application, it may be more preferably 950 MPa.

Tensile strength can be measured with an Amsler universal testing machine.

Further, torsional shear strength is required when screwing a screw, especially a medical screw. The torsional shear strength is roughly proportional to the hardness of the material.

Therefore, the Vickers hardness may be 200 HV or higher, preferably 220 HV or higher, more preferably 240 HV or higher.

Hardness can be measured with a Vickers hardness tester.

Furthermore, when used as a self-drilling screw, the surface hardness or external hardness and/or internal hardness (specifically, the hardness of the central portion and/or the hardness of the peripheral portion) measured with a Vickers hardness tester may be 250 HV or more, preferably 270 HV or more, specifically from 270 to 340 HV, more preferably 290 HV or more, specifically from 290 to 320 HV.

More, a screw is required to have high toughness (non-brittle property), and is generally required to have a sufficient necking at break, that is, to have a sufficient reduction of area. In particular, a high reduction in area is effective for the bending strength (resistance to bending breakage) required for the anchor screw.

Therefore, the reduction of area may be 45% or more, preferably 50% or more, and more preferably 60% or more, considering subsequent workability such as headability.

The term "reduction of area" used herein means plastic workability in the axial direction (longitudinal direction) of a substantially cylindrical shape.

The reduction of area can be measured by the evaluation value of the necking at the time of tensile breakage, and specifically can be tested and measured with an Amsler universal testing machine.

<Crystallite size>

In order to create a structure suitable for a screw, especially a medical screw, and in order to obtain the reliability of actual products, evaluation methods are important. Phenomena that occur in pure titanium result from a very complicated interplay of recrystallization, strain accumulation, crystal twinning and the like. Thus, since the crystal grains themselves become complicated, conventional evaluation methods such as measurement and evaluation using an optical microscope are difficult. Further, also in determining crystal grain size using transmission electron microscopy, complicated procedures such as setting the angle (inclination) are required. More, the method is not easily suitable as a process inspection/evaluation method during production.

Therefore, by using the crystallite size that exists as the original crystal unit in the complex crystal grains, an effective index is given to the above-mentioned complicated structure, and thus, screws of interest, in particular, medical screws have been found to have desirable mechanical properties.

A crystallite is a minimum unit that contributes to X-ray diffraction, unlike a crystal grain size, and is a portion of a crystal grain that can be regarded as a single crystal.

The crystallite size is a smaller value (or unit) than the crystal grain size determined from the apparent size of the crystal. In addition, in a pure single crystal, the grain size and the crystallite size can be considered to be almost the same, but if the crystal loses its regularity under various conditions due to processing, there is not necessarily a correlation between the grain size and the crystallite size.

It is decided to use the crystallite size as an index of whether pure titanium has desired properties, in particular mechanical properties, regardless of the presence or absence of processing and the processing ratio.

Crystallite size can be identified by X-ray diffraction (XRD) and can be also used as a production process check.

Specifically, the crystallite size can be measured as follows:
An X-ray diffractometer (D8 ADVANCE) manufactured by BRUKER is used, and K$\alpha$ rays of cobalt are used as X-rays. The output of the cobalt tube is 35 kV and the current is 40 mA.

In the present specification, the crystallite size is obtained by measuring the diffraction X-ray of the crystal plane (1 0 -1 0) of pure titanium.

The X-ray scanning range is $2\theta = 35.0°$ to $48.0°$, the divergence slit diameter is 0.3 mm, and the collimator diameter is 0.3 mm. Analysis of the measurement data is performed using BRUKER's analysis software DIFFRAC. EVA.

The crystallite size $L_{vol}$ can be obtained from the Scherrer equation represented by the following Equation 1, wherein the crystallite size is $L_{vol}$ [Å], the measurement wavelength is $\lambda$ [Å], the integrated width $\beta$ [rad] of the peak excluding the influence of the apparatus, and the angular position $\theta$ [rad] of the peak.

$$L_{vol} = \lambda \ / \ \beta \ \cos \ \theta \qquad\qquad (Equation\ 1)$$

Furthermore, for details of the crystallite size, refer to Waseda, Matsubara, Shinoda (2008), Basics of Exercise X-ray Structural Analysis (Uchida Rokakuho) pp. 103-108 (all of which are incorporated herein by reference).

According to the present invention, the crystallite size may be 280 Å or less, preferably 270 Å or less, more preferably 260 Å or less.

<Screw>

The screw according to the present invention may be formed from the base material for the screw described above. Therefore, the screw according to the present invention should have the same properties as the base material for the screw described above.

That is, the screw according to the present invention may be made of pure titanium and may have a substantially cylindrical shape, and the maximum value of the specific intensity of the orientation of the (1 0 -1 0) plane in the axial direction of the substantially cylindrical shape may be 3 or more, preferably 4 or more, more preferably 5 or more.

Further, the crystallite size of pure titanium may be 280 Å or less, preferably 270 Å or less, more preferably 260 Å or less.

More, the screw according to the present invention may have at least one, two, or three of the following mechanical properties i) to iii):

Property i): tensile strength of 800 MPa or more, preferably 860 MPa or more, more preferably 920 MPa or more;
Property ii): surface hardness (or external hardness) of 200 HV or more, preferably 220 HV or more, more preferably 240 HV or more; and
Property iii): reduction of area of 45% or more, preferably 50% or more, more preferably 60% or more.

Further, the screw according to the present invention may be made of pure titanium. The pure titanium may be selected from the group consisting of pure titanium Grade 2, pure titanium Grade 3, pure titanium Grade 4, and pure titanium with crystal grains refined to 1 μm or less, and the pure titanium may be preferably pure titanium Grade 4.

More, the screw according to the present invention may have a combination of A) the desired maximum value of the orientation specific intensity and B) the desired crystallite size. Alternatively, the screw according to the present invention may have a combination of B) desired crystallite size and C) at least one, two or three of mechanical properties i) to iii). Alternatively, the screw according to the present invention may have a combination of A) the desired maximum value of the orientation specific intensity, B) the desired crystallite size, and C) the at least one, two or three of mechanical properties i) to iii).

The base material for screws according to the present invention may be a base material for a medical screw, in particular a base material for a medical anchor screw. The base material for the medical anchor screw may be a base material for an orthodontic anchor screw.

Further, the screw according to the present invention may be a medical screw, in particular a medical anchor screw. The medical anchor screw may be an orthodontic anchor screw.

In the screw according to the present invention, an aspect ratio of the titanium crystals in the central part in a plane parallel to the axial direction of the screw and the plane including the axis may be larger than an aspect ratio of the titanium crystals in the peripheral part. The aspect ratio of the central part may preferably be 1.10 to 1.50 times, more preferably 1.20 to 1.50 times, most preferably 1.30 to 1.50 times larger than the aspect ratio of the peripheral part.

The screw having the above configuration can exhibit the effect of Functionally Graded Materials (commonly known as FGM's).

In this specification, the aspect ratio of a titanium crystal can be determined by measuring an EBSD pattern (Electron Back-Scattered Diffraction Pattern), approximating the titanium crystal to an ellipse, and taking the major axis/minor axis as the aspect ratio.

Specifically, the EBSD pattern is measured using a crystal orientation analysis detector manufactured by EDAX (model: EDS/EBSD integration system Pegasus) attached to a Hitachi High-Tech FESEM (Field Emission Scanning Electron Microscope, model: SU5000), and the EBSD pattern can be analyzed using crystal orientation analysis software manufactured by EDAX (model: OIM Analysis v8).

More specifically, SEM images are observed at an acceleration voltage of 15 kV and an emission current of 70 $\mu$A, and EBSD patterns are measured in an area of 10 $\mu$m × 10 $\mu$m with a step width of 0.1 $\mu$m, and analyzed after background removal. A difference of 5° or more in the crystal orientation between each measured pixel is regarded as a grain boundary, and analysis parameters are set so that an area containing at least five or more connected pixels is recognized as a crystal grain. The identified crystal grain is approximated as an ellipse, and its major axis/minor axis is taken as the aspect ratio.

The screw according to the present invention may be a self-drilling screw, and the surface or external hardness, and/or internal hardness (specifically, the hardness of the central part and/or the hardness of the peripheral part) may be 250 HV or more, preferably 270 HV or more, specifically from 270 to 340 HV, more preferably 290 HV or more, specifically from 290 to 320 HV.

<Producing method of a base material for a screw >

The base material for the screw can be produced by the following producing method:
The method comprises the steps of:

    (Ia) preparing a pure titanium material; and
    (IIa) swaging the pure titanium material; to obtain the base material for the screw.

In particular, the method consists of the steps of:

    (Ia) preparing a pure titanium material; and
    (IIa) swaging the pure titanium material; to obtain the base material for the screw.

The step of processing pure titanium material adopts "swaging", and the reason why the step adopts "swaging" is as follows:
Deformation (plastic deforming) of the metal material converts approximately 90% of the strain energy introduced by the deformation into heat (processing heat), thereby increasing the temperature of the metal material itself. In general, the heat generated during processing is considered to around 100°C, depending on the processing method. Therefore processing does not utilize the heat generated solely during processing. On the contrary, molds are cooled during processing, in order to suppress the disadvantage of the heat generated.

Titanium has a low thermal conductivity, which suppresses the diffusion of heat throughout the titanium material, and the heat stays in the part where the material undergoes plastic deformation, and thus a temperature of titanium material rises more. If the processing conditions are the same, the thermal conductivity of titanium is less than half that of steel, and thus, it is thought that the temperature of the processed portion of titanium material will rise about twice than that of iron material, that is, around 200°C.

Further, increased processing speed (or strain rate) leads to the high-speed processing region above a certain speed, and can locally generate a large amount of heat through a mechanism different from the above-described processing heat. In swaging, for example, it is also possible to achieve impact processing speeds of the surface of approximately 50 meters/second, because of the availability of high speed rotation of the peripheral rollers. The strain rate at that time is about 10 to 100/s, which is almost in the region of high-speed processing.

If the contact time between the mold and the titanium material is instantaneous, there is almost no heat flow to the mold, and thus the heat is trapped inside the material. Swaging has such properties, so that swaging is a preferred processing method, which increases the temperature of the material itself by the heat generated due to processing.

In this way, the combination of titanium's low thermal conductivity and high-speed processing can raise the temperature of the titanium material to 300°C or higher without heating up from the surroundings. Specifically, properly setting of the rotation speed for high-speed processing, the insertion speed of the workpiece, the contact time with the die, the amount of lubricant applied, the processing ratio per time (the processing ratio in a single process), and the total processing ratio (total of the processing ratio in a single process) can control the strain speed while maintaining the internal temperature of the material at 300 to 400°C, making it possible to create an appropriate balanced state (equilibrium state) between the introduction of processing strain and the generation of recrystallization.

By utilizing the processing heat of the material itself in this way, it is possible to control the state of the crystals and their orientation, and the strength and toughness of the material, to form an appropriate structure for the screw having the above-described properties.

According to this method, it is possible to continue processing without performing intermediate annealing (softening heat treatment to give ductility to the material), up to the final material diameter by reducing the material diameter by repeating the single process several times to several tens of times. In this way, since the ductility is spontaneously improved by recrystallization that occurs during processing, it is possible to increase the total processing ratio (sum of single steps) to 80 to 95% or more beyond the conventional case.

Since it is possible to obtain an extremely high total processing ratio, it is possible to absorb variations in the state of the structure of the material before processing.

When such a method is used, it is possible that desired properties can be imparted to the medical screw even by using a bar or wire made of pure titanium Grades 1 to 4 without using a material that has been grain refining, such as a material made by repeated shear deformation processing (ECAP) or multi-axis forging (MDF), as a pre-processing material.

Specifically, the present invention provides the following producing method:

The present invention provides a method for producing a base material for a screw, comprising the steps of:

(I) preparing a pure titanium material having a substantially cylindrical shape and a cross-sectional area of A0; and
(II) swaging the pure titanium material;

to obtain the base material for the screw, which has a substantially cylindrical shape having a cross-sectional area of A1 after swaging and a true strain represented by ln(A0/A1) of 2 or more, preferably 2.5 or more, more preferably 3.0 or more, wherein the maximum value of the specific intensity of the orientation of the (1 0 -1 0) plane in the axial direction of the substantially cylindrical shape is 3 or more, preferably 4 or more, more preferably 5 or more, and a hardness of a central part and a peripheral part in a substantially circular cross section perpendicular to the axial direction are substantially same, and preferably the difference between the hardness of the central part and the peripheral part is 15 HV or less, more preferably 12 HV or less, and most preferably 10 HV or less.

In particular, the method for producing a base material for a screw consists of the steps of:

(I) preparing a pure titanium material having a substantially cylindrical shape and a cross-sectional area of A0; and
(II) swaging the pure titanium material;

to obtain the base material for the screw, which has a substantially cylindrical shape having a cross-sectional area of A1 after swaging and a true strain represented by ln(A0/A1) of 2 or more, preferably 2.5 or more, more preferably 3.0 or more, wherein the maximum value of the specific intensity of the orientation of the (1 0 -1 0) plane in the axial direction of the substantially cylindrical shape is 3 or more, preferably 4 or more, and more preferably 5 or more, and a hardness of a central part and a peripheral part in a substantially circular cross section perpendicular to the axial direction are substantially same, and preferably the difference between the hardness of the central part and the peripheral part is 15 HV or less, more preferably 12 HV or less, and most preferably 10 HV or less.

The term "true strain" used herein means an index indicating the processing ratio, and the true strain $\varepsilon$ can be expressed by the following equation 2 from the cross-sectional area A0 before processing and the cross-sectional area A1 after processing.

$$\varepsilon = \ln\left(\frac{A_0}{A_1}\right) \qquad \text{(Equation 2)}$$

The term "processing ratio" used herein means literally an index indicating the processing ratio, and the processing ratio e can be expressed by the following equation 3 from the cross-sectional area A0 before processing and the cross-sectional area A1 after processing.

$$e = \frac{A_0 - A_1}{A_0} \times 100 \qquad \text{(Equation 3)}$$

For example, when the processing ratio e is 80%, the true strain $\varepsilon$ is 1.61, when the processing ratio e is 90%, the true strain $\varepsilon$ is 2.3, and when the processing ratio e is 95%, the true strain $\varepsilon$ is 3.0.

According to the present invention, the true strain $\varepsilon$ may be 2 or more (processing ratio of 86% or more), preferably 2.5 or more (processing ratio of 92% or more), more preferably 3 or more (processing ratio of 95% or more).

The base material for the screw obtained by the method according to the present invention has the same definition and the same properties as described above.

The swaging conditions are not particularly limited as long as the above-described true strain $\varepsilon$ and/or the above-described processing ratio e can be achieved.

For example, swaging conditions may include, but are not limited to, setting conditions such that the surface temperature of the workpiece being worked is 250°C or higher.

In the manufacturing method according to the present invention, since the micromachining essentially consists of swaging or consists of swaging alone, the grain flow lines of the obtained base material and the screw manufactured from the base material are approximately parallel to the central axis of the material both during and after processing. Therefore, any part of the obtained base material or screw, for example in the longitudinal direction, or for example in the central part and the peripheral part, can have the same characteristics. Also, the variation between lots is reduced. Further, the method according to the present invention makes it possible to control the crystallite size and crystal orientation (orientation) of pure titanium, and to provide the above-mentioned crystallite size and orientation.

Further, the present invention also provides a method for producing a screw.

The present invention also provides a method for producing a screw, the method further comprising the step of: (III) imparting a screw shape to the base material for the screw obtained by the above-described method for producing the base material for the screw or the base material for the screw having the above-described properties; to obtain the screw.

In the above-described producing method, the terms "true strain", "processing ratio", the oxygen content of pure titanium material used, the oxygen content in the obtained base material or the obtained screw, and the crystallite size of the pure titanium in the obtained base material or the obtained screw have the same definitions as described above.

In the step (III), it is preferable to give the screw shape by cutting the base material for the screw having the substantially cylindrical shape.

By obtaining the screw by cutting, the surface temperature is preferentially increased, making it possible to provide a screw in which the aspect ratio of the titanium crystals in the central part in a plane parallel to the axial direction of the screw and including the axis is larger than in that of the peripheral part, and the aspect ratio of the central part may be preferably 1.10 to 1.50 times, more preferably 1.20 to 1.50 times, most preferably 1.30 to 1.50 times larger than that of the peripheral part.

The method for producing the screw may further comprise a step of (IV) pressing the base material for the screw at 250°C or less, to form the screw head. The step (IV) may be performed before or after the step (III).

Examples:

<Pure titanium material>

As pure titanium materials, i) pure titanium Grade 2 with a wire diameter of 5.8 mm (CP-T2) (manufactured by Toho Tech Co., Ltd.), and ii) pure titanium Grade 4 with a wire diameter of 6.0 mm (CP-T4) (manufactured by Toho Tech Co., Ltd.) were prepared. Further, iii) a block-shaped material (manufactured by Kawamoto Heavy Industries, Ltd.) was prepared by refining a pure titanium Grade 2 with crystals to less than 1 $\mu$m meter by bulk ultrafine grained processing (UFG), and the block-shaped material was cut out, to prepare a bar material (FTi2) with a wire diameter of 6.0 mm.

These materials were swaged several times at room temperature using a 15HP-SD type (four-way) swaging device manufactured by Yoshida Kinen Co., Ltd. The true strain of each single swaging step was set to 0.15 to 0.25, followed by repeating the step several times, to prepare four to five processed base materials each having a rod shape with the true strain ranging from 0 to about 3.7 in total (processing ratio: about 97%).

Furthermore, in each swaging step, the surface temperature of the material was measured using a radiation thermometer, to adjust the surface temperature, swager rotation speed, bar material advance speed, lubrication so that the temperature was 300 to 400 °C, and the application of oil per hour. The strain rate was calculated from the swager conditions described above.

For the rod-shaped processed base materials obtained by swaging FTi2 and CP-T4, the oxygen content was that provided by the manufacturer.

The surface hardness (or external hardness) was measured by a micro Vickers hardness tester under a load of 2.94 N. When the surface was curved, the curved surface was slightly scraped to make it flat, and the indenter was pressed into the flat surface to measure the Vickers hardness.

The results are shown in Table 1.

Table 1. Oxygen contents and Vickers hardness of FTi2 and CP-T4 before and after swaging

|  | FTi2 | | CP-T4 | |
|---|---|---|---|---|
| Oxygen contents (wt%) | 0.12 | 0.14 | 0.28 | 0.34 |
| Before swaging (HV) | 156 | 165 | 208 | 220 |
| After swaging (HV) | 225 | 240 | 289 | 308 |

<Crystallite size and orientation>

Samples for X-ray diffraction for determining the crystallite size and orientation were obtained by cutting each processed base material having the rod shape in a plane perpendicular to the axial direction and embedding it in a phenolic resin. For the resin-filled sample, wet polishing was performed through SiC waterproof abrasive paper #400, #800, #1200, and #2400 in order from the rough side so that the surface was exactly perpendicular to the axial direction of each base material. Then, each sample was buffed with a silicon dioxide suspension (OP-S) to give a mirror finish.

The crystallite size was measured using a BRUKER X-ray diffractometer (D8 ADVANCE) with cobalt K$\alpha$ rays at a cobalt tube output of 35 kV and a current of 40 mA, with the conditions: X-ray scanning range 2$\theta$: 35.0° to 48.0°, the divergence slit diameter: 0.3 mm, and the collimator diameter: 0.3 mm. The measurement data were analyzed using analysis software DIFFRAC. EVA (manufactured by BRUKER).

Orientation was measured using an X-ray diffractometer (D8 ADVANCE) manufactured by BRUKER under the same conditions as described above.

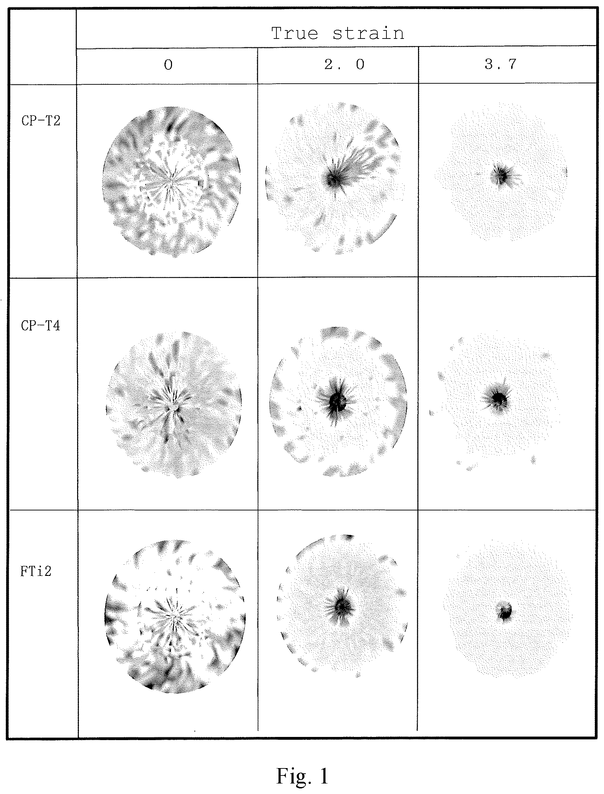

The in-plane direction angle $\Phi$ of the sample was measured in 72 steps in 5-degree increments around 360 degrees, and the range of the tilt angle $\Psi$ was measured with a starting point of 15 degrees and an end point of 45 degrees. The resulting data were analyzed using analysis software DIFFRAC. TEXTURE MRDB V4.1 (manufactured by BRUKER), to create a pole figure of the (1 0 -1 0) plane. The pole figure is shown in Fig.1.

The maximum value of the relative intensities where the average intensity of the entire pole figure was defined as 1 was defined as the maximum specific intensity. Furthermore, in Fig. 1, in a case where the material is isotropic, that is, in a case where there is no orientation, there is little color shading. In a case where orientation appears, a dark area appears at a certain degree, and the relative intensity at the angle increases.

For the resulting processed base materials each having a rod shape, the crystallite size and the orientation of the (1 0 -1 0) plane of pure titanium in the axial direction were obtained under the conditions described above.

<Mechanical properties>

Mechanical properties such as tensile strength, Vickers hardness, and reduction of area were measured for the resulting processed base materials each having a rod shape.

The tensile test was carried out with an Amsler universal testing machine.

Surface hardness (or external hardness) was measured with a micro Vickers hardness tester under a load of 2.94 N.

Further, the internal hardness (the hardness of the central part and the peripheral part in a substantially circular cross section perpendicular to the axial direction of the rod-shaped base material) was measured as described above.

More, the reduction of area (RA) was obtained by converting the diameter of the sample after breakage in the tensile test into an area according to Equation 4, wherein $D_0$ means the diameter of the material before tensile testing and $D_1$ means the diameter of the neck of the material after tensile testing.

$$\mathrm{RA} = \frac{D_0^2 - D_1^2}{D_0^2} \times 100 \qquad \text{(Equation 4)}$$

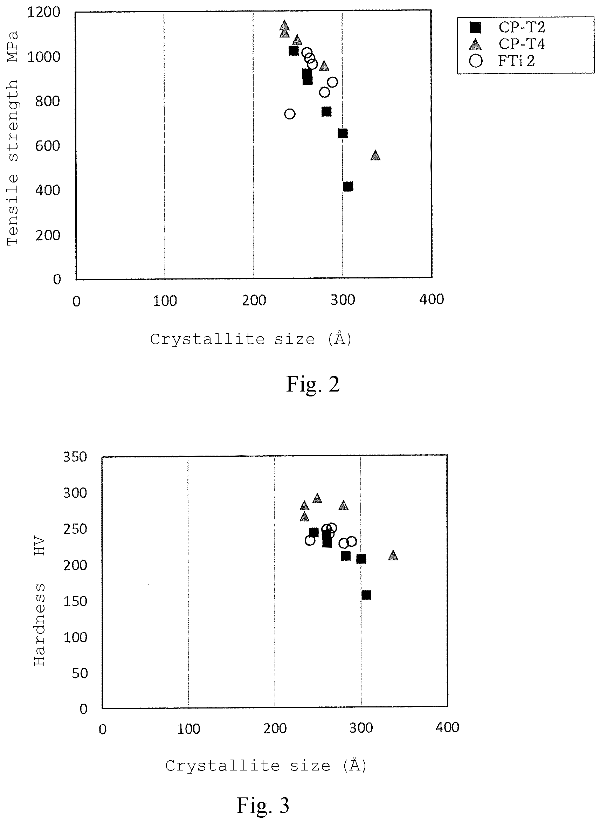

Figs. 2 to 4 show graphs in which the horizontal axis is the crystallite size and the vertical axis is the mechanical properties.

Specifically, Fig. 2 shows a graph in which the horizontal axis is the crystallite size and the vertical axis is the tensile strength.

Further, Fig. 3 shows a graph in which the horizontal axis is the crystallite size and the vertical axis is the hardness.

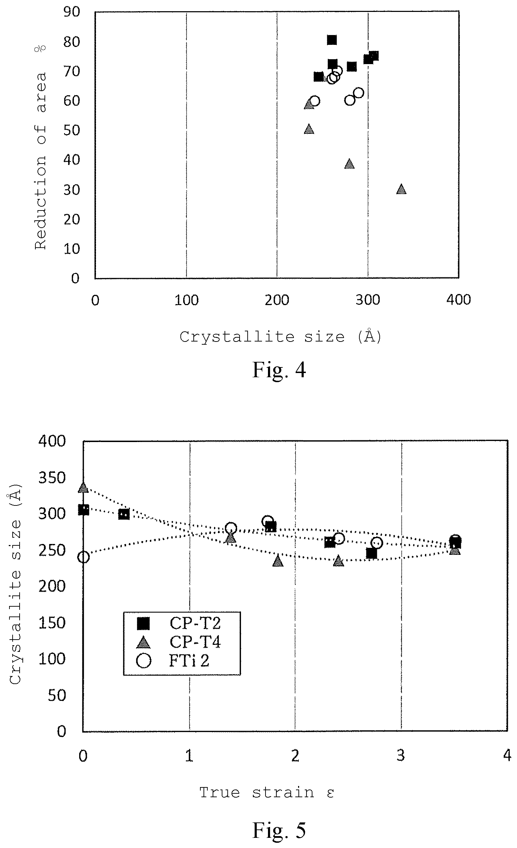

More, Fig. 4 shows a graph in which the horizontal axis is the crystallite size and the vertical axis is the reduction of area.

Figs. 2 to 4 show that in a case where the base material has desired mechanical properties, for example, tensile strength of 800 MPa or more, hardness of 200 HV or more, and reduction of area of 45% or more, the crystallite size is 280 Å or less.

Therefore, Figs. 2 to 4 show that pure titanium having a crystallite size of 280 Å or less provides the base material having desired mechanical properties.

Fig. 5 shows a graph in which the horizontal axis is the true strain and the vertical axis is the crystallite size.

Fig. 5 shows that the true strain should be 2 or more (processing ratio: 86% or more), in order to make the crystallite size 280 Å or less.

Further, the results of Fig 5 together with the results of Figs. 2 to 4 show that a base material having desired mechanical properties can be obtained by setting the true strain to 2 or more (processing ratio: 86% or more).

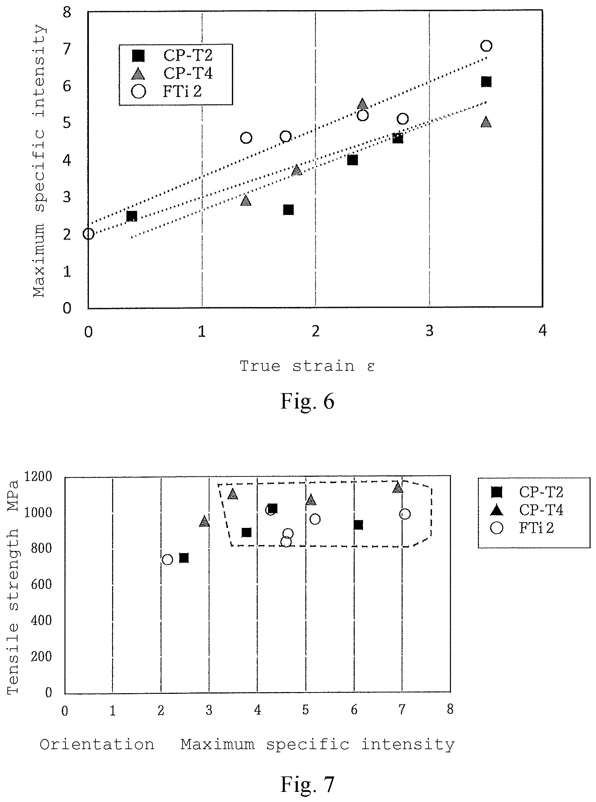

Fig. 6 shows a graph in which the horizontal axis is the true strain and the vertical axis is the maximum specific intensity.

Fig. 6 shows that the true strain should be 2 or more in order to obtain a material having a maximum specific intensity of 3 or more.

Figs. 7 to 9 show graphs in which the horizontal axis is the maximum specific intensity and the vertical axis is the mechanical property.

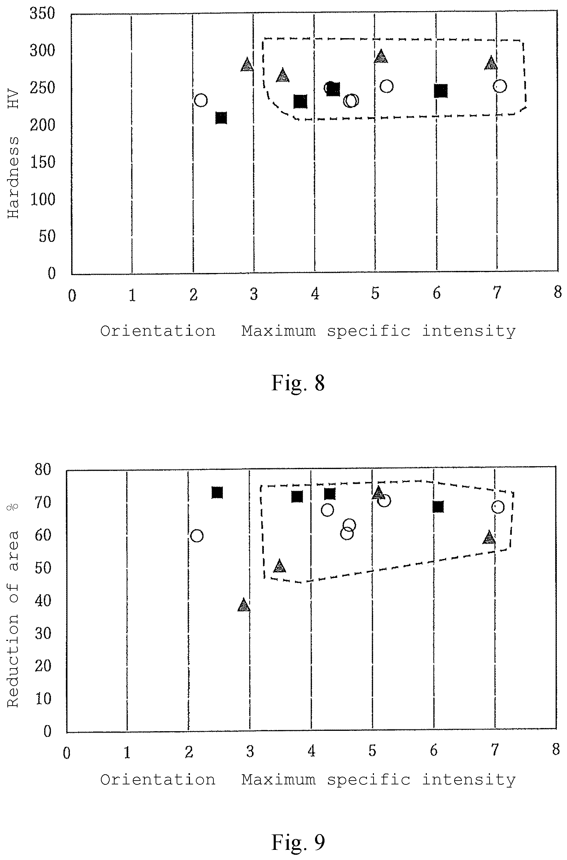

Specifically, the vertical axis of Fig. 7 is tensile strength, the vertical axis of Fig. 8 is hardness, and the vertical axis of Fig. 9 is reduction of area.

Figs. 7 to 9 show that in a case where the maximum specific intensity is 3 or more, desired mechanical properties such as tensile strength of 800 MPa or more, surface hardness of 200 HV or more, and reduction of area of 45% or more are obtained.

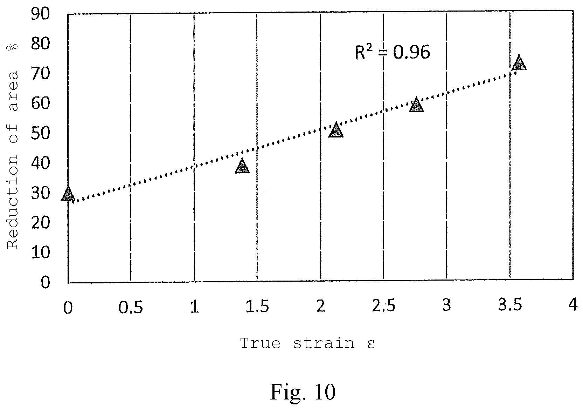

Fig. 10 shows a graph showing the relationship between true strain (horizontal axis) and reduction of area of a base material obtained using material CP-T4.

Fig. 10 shows that the reduction of area increases as the true strain increases. In particular, Fig. 10 shows that the reduction of area increases to 70% or more at a true strain of 3.5 (processing ratio: 97%). Furthermore, the value is a value corresponding to pure titanium Grade 2.

Further, Fig. 10 shows that in a case where the true strain is 2 or more, the reduction is 45% or more.

Figs. 10 and 5 show that the swaging process should be performed so that the true strain is 2 or more (processing ratio: 86% or more).

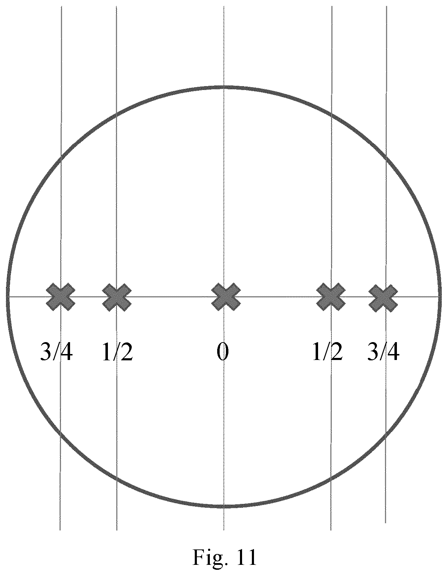

Fig. 11 shows a diagram showing the points where the "internal hardness" was measured in a substantially circular cross section perpendicular to the axial direction of the rod-shaped processed base material. In the figure, the point represented by "0" is the point where the hardness of the central part was measured, and the point represented by "3/4" is the point where the hardness of the peripheral part was measured at a position of 3/4 of the radius. Furthermore, the point represented by "1/2" is a point between the central part and the peripheral part at a position of 1/2 of the radius, and the hardness was measured between the central part and the peripheral part other than the central and peripheral parts.

Table 2 shows the Vickers hardness measured at each point in Fig. 11. Table 2 shows that in the substantially circular cross section perpendicular to the axial direction of the rod-shaped processed base material, the hardness of the central part (point "0") is 297 HV, and the hardness of the peripheral part (point "3/4") is 304 HV or 305 HV, with a difference of 8 HV, which is substantially same.

Table 2. Hardness of the central and the peripheral parts of the approximately circular cross section perpendicular to the axial direction of the rod-shaped processed base material

| Measured point in Fig. 11 | Vickers hardness (HV) |
|---|---|
| 3/4 | 305 |
| 1/2 | 299 |
| 0 | 297 |
| 1/2 | 300 |
| 3/4 | 304 |

<Head formability>

Table 3 shows the head formability of the screw formed using the processed base materials each having a rod shape.

Furthermore, it should be noted that the screw could be formed from the processed base material having the rod shape by upsetting (heading) like normal screw forming.

Table 3 shows that using ii) CP-T4 as the raw material, the processed base material having the rod shape obtained with a true strain of 2.65 (processing ratio: 93%) or more could be obtained at a temperature of 200°C, which is lower than that of 400°C or higher heading processing was usually performed in the vicinity of true strain: 0 (processing ratio: 0%).

Table 3.

| | True strain (Processing ratio %) | Processing temperature (°C) | | | | | |
|---|---|---|---|---|---|---|---|
| | | 100 | 150 | 200 | 250 | 300 | 350 |
| CP-T4 | 0 (0%) | × | × | × | × | △ | ○ |
| | 2.0(86%) | × | × | ○ | ○ | ○ | ○ |
| | 2.65(93%) | × | × | ○ | ○ | ○ | ○ |
| FTi2 | 0 (0%) | × | × | ○ | ○ | ○ | ○ |
| | 2.65(93%) | × | × | ○ | ○ | ○ | ○ |

○: Stable processing is possible; △: Processing is possible;
×: Cracks in head

Conventionally, a medical screw made of pure titanium Grade 4, which have a high oxygen content among pure titanium, has high strength but poor plastic workability. Thus, it was difficult to heading process the medical screw at a temperature of 200°C or less. Therefore, the head of the screw was formed by hot working (400 °C or higher) or cutting. Machining in these temperature ranges required solid lubrication, which significantly reduced productivity. In order to reduce costs and to improve productivity, there has been a demand for the same material that has plastic workability (especially reduction of area) that allows heading at 250 °C or lower.

However, as can be seen from Table 3, according to the present invention, head processing by heading is possible even at 250 °C or less.

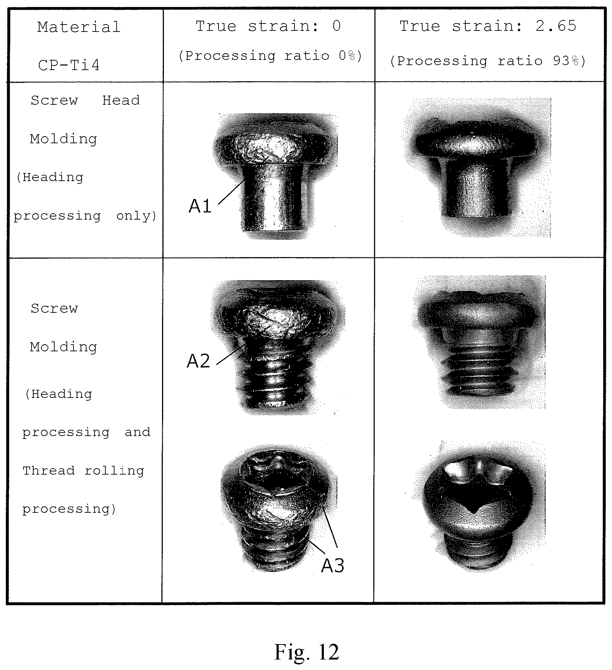

Fig. 12 shows a photograph of the head of CP-T4 when the recess on the head was processed into a hexalobular shape. In a case where CP-T4 was used without processing (true strain: 0, processing ratio: 0%), cracks (ductile fracture) occurred in the head of the screw at 200 °C, as shown by A1, A2 and A3 in Fig 11, and thus head molding could not be performed. However, using ii) CP-T4 as the material, the processed base materials having the rod shape obtained at a true strain of 2 (processing ratio: 86%) and a true strain of 2.65 (processing ratio: 93%) were possible to process normally without cracks.

The rod-shaped processed base material obtained above was cut to give it a screw shape. Specifically, a screw was formed by turning using a carbide cutting tool while applying a constant flow rate of cutting oil.

The aspect ratio of the titanium crystal in the axial direction of the screw, i.e., the plane parallel to the longitudinal direction of the screw and including the screw axis, of the screw obtained by this was measured by measuring the EBSD pattern using a crystal orientation analysis detector (model: EDS/EBSD integration system Pegasus) manufactured by EDAX attached to a Hitachi High-Tech FESEM (Field Emission Scanning Electron Microscope, model: SU5000), and analyzing it using crystal orientation analysis software (model: OIM Analysis v8) manufactured by EDAX. More specifically, the SEM image was observed at an acceleration voltage of 15 kV and an emission current of 70 μA, and the EBSD pattern was measured in an area of 10 μm × 10 μm with a step width of 0.1 μm, and analyzed after background removal. The analysis parameters were set so that a grain boundary was recognized when there was a difference of 5° or more in the crystal orientation between each measured pixel, and an area in which at least five or more connected pixels exist was recognized as a crystal grain. The identified crystal grains were approximated as ellipses, and the major axis/minor axis ratio was taken as the aspect ratio.

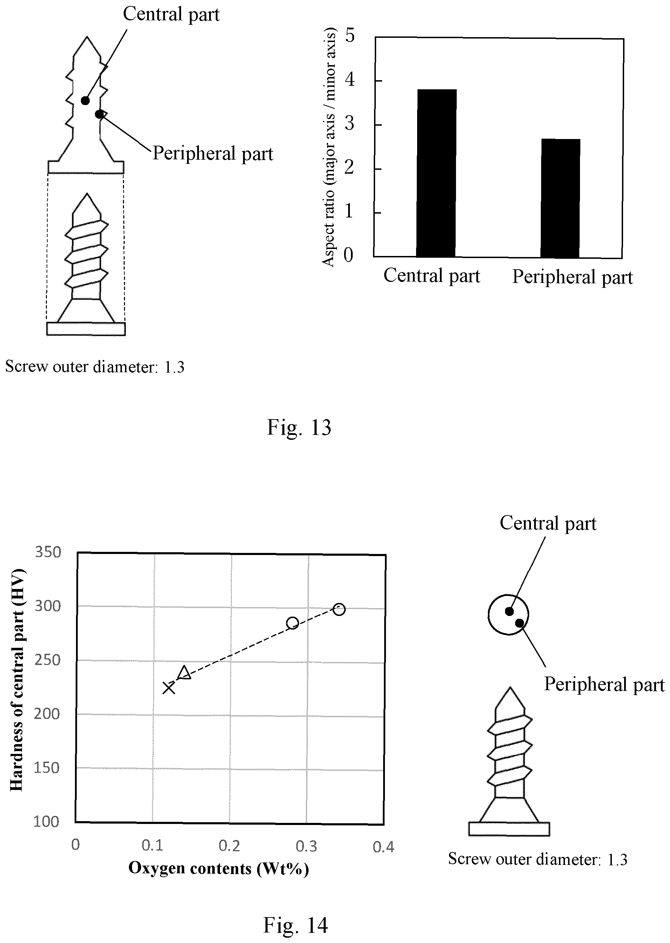

Fig. 13 shows a schematic diagram of the measured surface and the locations of the measured screw, as well as the aspect ratio results obtained.

Figure 13 shows that the aspect ratio of the central part was 3.82, while that of the peripheral part was 2.71. This shows that the grain size of the crystals in the peripheral part was increased, improving the torsional strength of the screw. In addition, the peripheral part was preferentially heated by the processing heat generated on the surface by cutting the base material into the screw, which removed the distortion in this area, improving the wear resistance and providing the screw with the characteristics of a self-drilling screw.

\<Hardness, oxygen content, and self-drilling\>

The hardness (of the central and peripheral parts) of the screws obtained using ii) CP-T4 as the material (oxygen content: 0.28 % by weight and 0.34 % by weight) and the screws obtained using FTi2 (oxygen content: 0.12 % by weight and 0.14 % by weight) was measured. The results are shown in Fig. 14, where the horizontal axis is the oxygen content and the vertical axis is the hardness of the central part.

The screw used was a standard anchor screw shape with a threaded portion having an outer diameter of 1.3 mm and a length of 6 mm, and the cutter at the tip, which serves as a drill, was a standard single-cut one with a notch.

The hardness of the central and peripheral parts of the screw (oxygen content: 0.34 % by weight) obtained using CP-T4 was measured. As a result, it was found that the hardness of the central part was 299 HV and the hardness of the peripheral part was 295 HV, a difference of 4 HV. These results show that even when made into the screw, the hardness of the central and the peripheral parts are approximately same in an approximately circular cross section perpendicular to the axial direction of the screw, just like the results of the hardness of the central and the peripheral parts of the base material in Table 2 above.

We also investigated whether these screws could be used as self-drilling screws. The results of the investigation are shown in Fig. 14 as "○", "△" and "×". Specifically, we used a simulated bone (Sawbones solid rigid polyurethane 20pct, 2cm square block) as the subject and self-drilled the screws, obtaining the following results.

That is, the screw having an oxygen content of 0.12 % by weight had a hardness of about 225 HV at the central part. In addition, when five screws having an oxygen content of 0.12 wt% were used, the screw bodies of four out of the five screws broke (indicated as "x" in Fig. 14).

Further, the hardness of the central part of the screw having an oxygen content of 0.14 % by weight was about 240 HV. When five screws having an oxygen content of 0.14 % by weight were used, two screw bodies among the five screws broke (indicated as "△" in Fig. 14).

On the other hand, the screws each having the oxygen content of 0.28 % by weight and 0.34 % by weight had the hardness of the central part of approximately 290 HV and 310 HV, respectively. When five of each of these screws were used, implantation was completed successfully for all five screws (indicated as "O" in Fig. 14).

\<Torsional breaking strength, oxygen content, and selfdrilling\>

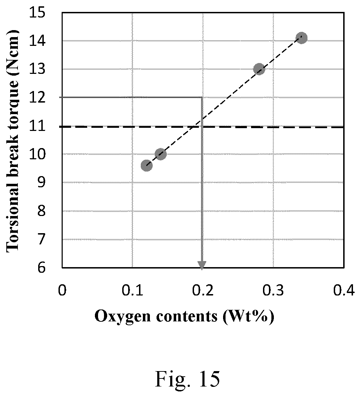

As in \<Hardness, oxygen content, and self-drilling\>, ii) the torsional breaking strength was measured for screws obtained using CP-T4 (oxygen content: 0.28 % by weight and 0.34 % by weight) and screws obtained using FTi2 (oxygen content: 0.12 % by weight and 0.14 % by weight). The results are shown in Fig. 15, where the horizontal axis is the oxygen content and the vertical axis is the torsional strength.

Fig. 15 shows that the screw having the oxygen content of 0.12 % by weight had a torsional breaking strength of about 9.6 Ncm. Also, as described above, when five screws each having the oxygen content of 0.12 % by weight were used, four screw bodies among the five screws broke.

Fig. 15 shows that the screw having the oxygen content of 0.14 % by weight had a torsional fracture strength of about 10 Ncm. Also, as described above, when five screws each having the oxygen content of 0.14 % by weight were used, two screw bodies among the five screws broke.

On the other hand, the screws each having the oxygen content of 0.28 % by weight and 0.34 % by weight had torsional breaking strengths of about 13 Ncm and 14 Ncm, respectively. When five of each of these screws were used, implantation was completed normally for all five.

These findings show that a self-drilling screw can be provided by using a screw having a certain amount of oxygen or more.

According to the Orthodontic Anchor Screw Guidelines (Japanese Society of Orthodontics, 2012), a torsional break torque of 11 Ncm or more is recommended for anchor screws. In Fig. 15, in order to meet the torsional break torque of 11 Ncm or more, an oxygen content of 0.2% or more is required. Also, Fig. 14 shows that the oxygen content of 0.2% or more results in a hardness of HV250 or more. In other words, Fig. 14 shows that a hardness, specifically a surface hardness or internal hardness (hardness of the central and/or the peripheral parts) of 250 HV or more, preferably 270 HV or more, specifically from 270 to 340 HV, more preferably 290 HV or more, specifically from 290 to 320 HV, can be used as a self-drilling screw.

**Claims**

1. A base material for a screw having a substantially cylindrical shape or a screw having a substantially cylindrical shape, wherein each of the base material and the screw is made of pure titanium, each of the base material and the screw has 3 or more of a maximum specific intensity of the orientation in the (1 0 -1 0) plane in the axial direction of the substantially cylindrical shape, and a hardness of a central part and a hardness of a peripheral part in a substantially circular cross section perpendicular to the axial direction are substantially same.

2. The base material or the screw according to claim 1, wherein an oxygen content in the base material or the screw is 0.2 to 0.4 % by weight, when the total weight of the base material or the screw is normalized as 100 % by weight.

3. The base material or the screw according to claim 1 or 2, wherein the crystallite size of the pure titanium is 280 Å or less.

4. The base material or the screw according to any one of claims 1 to 3, wherein each of the base material and the screw has at least one of the following properties i) to iii) :

   Property i): tensile strength of 800 MPa or more;
   Property ii): surface hardness of 200 HV or more; and
   Property iii): reduction of area of 45% or more.

5. The base material or the screw according to any one of claims 1 to 4, wherein the pure titanium is selected from the group consisting of pure titanium Grade 2, pure titanium Grade 3, pure titanium Grade 4, and pure titanium with crystal grains refined to 1 $\mu$m or less.

6. The base material or the screw according to any one of claims 1 to 5, wherein the pure titanium is pure titanium Grade 4.

7. The base material or the screw according to any one of claims 1 to 6, wherein the base material or the screw is a screw, and an aspect ratio of the titanium crystals in the central part in a plane parallel to the axial direction of the screw and the plane including the axis is larger than an aspect ratio of the titanium crystals in the peripheral part.

8. The base material or the screw according to any one of claims 1 to 7, wherein the base material or the screw is a screw, and the screw has a surface hardness and/or an internal hardness of 250 HV or more.

9. The base material or the screw according to any one of claims 1 to 8, wherein the base material or the screw is a base material for a medical anchor screw or a medical anchor screw.

10. The base material or the screw according to any one of claims 1 to 9, wherein the base material or the screw is a base material for an orthodontic anchor screw or an orthodontic anchor screw.

11. A method for producing a base material for a screw, comprising the steps of:

   (I) preparing a pure titanium material having a substantially cylindrical shape and a cross-sectional area of A0; and
   (II) swaging the pure titanium material, to obtain the base material for the screw, which has a substantially cylindrical shape having a cross-sectional area of A1 after swaging and a true strain represented by ln(A0/A1) of 2 or more, wherein the maximum value of the specific intensity of the orientation of the (1 0 -1 0) plane in the axial direction of the substantially cylindrical shape is 3 or more, and a hardness of a central part and a peripheral part in a substantially circular cross section perpendicular to the axial direction are substantially same.

12. A method for producing a base material for a screw, consisting of the steps of:

   (I) preparing a pure titanium material having a substantially cylindrical shape and a cross-sectional area of A0; and
   (II) swaging the pure titanium material, to obtain the base material for the screw, which has a substantially cylindrical shape having a cross-sectional area of A1 after swaging and a true strain represented by ln(A0/A1) of 2 or more, wherein the maximum value of the specific intensity of the orientation of the (1 0 -1 0) plane in the axial direction of the substantially cylindrical shape is 3 or more, and a hardness of a central part and a peripheral part in a substantially circular cross section perpendicular to the axial direction are substantially same.

13. A method for producing a screw, further comprising the step of (III) imparting a screw shape to the base material for the screw having the substantially cylindrical shape obtained in the method according to claim 11 or 12; to obtain the screw.

14. The method according to claim 13, wherein the screw shape is imparted by cutting the base material for the screw having the substantially cylindrical shape.

15. The method according to claim 13 or 14, further comprising the step of (IV) molding the base material for the screw having the substantially cylindrical shape at 250 °C or less, to form a screw head.

16. The method according to any one of claims 11 to 15, wherein an oxygen content in the base material or the screw is 0.2 to 0.4 % by weight, when the total weight of the base material or the screw is normalized as 100 % by weight.

17. The method according to any one of claims 11 to 16, wherein the crystallite size of the pure titanium is 280 Å or less.

18. The method according to any one of claims 11 to 17, wherein the crystallite size of the base material or the screw each having the substantially cylindrical shape is 280 Å or less.

19. The method according to any one of claims 11 to 18, wherein each of the base material and the screw has at least one of the following properties i) to iii):

   Property i): tensile strength of 800 MPa or more;
   Property ii): surface hardness of 200 HV or more; and
   Property iii): reduction of area of 45% or more.

20. The method according to any one of claims 11 to 19, wherein the pure titanium is selected from the group consisting of pure titanium Grade 2, pure titanium Grade 3, pure titanium Grade 4, and pure titanium with crystal grains refined to 1 μm or less.

21. The method according to any one of claims 11 to 20, wherein the pure titanium is pure titanium Grade 4.

22. The method according to any one of claims 11 to 21, wherein the base material or the screw is a screw, and an aspect ratio of the titanium crystals in a central part in a plane parallel to the axial direction of the screw and the plane including the axis is larger than an aspect ratio of the titanium crystals in a peripheral part.

23. The method according to any one of claims 11 to 22, wherein the base material or the screw is a screw, and the screw has a surface hardness and/or an internal hardness of 250 HV or more.

24. The method according to any one of claims 11 to 23, wherein the base material or the screw is a base material for a medical anchor screw or a medical anchor screw.

25. The method according to any one of claims 11 to 24, wherein the base material or the screw is a base material for an orthodontic anchor screw or an orthodontic anchor screw.

| | True strain | | |
|---|---|---|---|
| | 0 | 2.0 | 3.7 |
| CP-T2 | | | |
| CP-T4 | | | |
| FTi2 | | | |

Fig. 1

Fig. 2

Fig. 3

EP 4 635 523 A1

Fig. 4

Fig. 5

22

Fig. 6

Fig. 7

Fig. 8

Fig. 9

Fig. 10

Fig. 11

| Material<br><br>CP-Ti4 | True strain: 0<br>(Processing ratio 0%) | True strain: 2.65<br>(Processing ratio 93%) |
|---|---|---|
| Screw  Head<br><br>Molding<br><br>(Heading<br><br>processing  only) | | |
| Screw<br><br>Molding<br><br>(Heading<br><br>processing  and<br><br>Thread rolling<br><br>processing) | | |

Fig. 12

Screw outer diameter: 1.3

Fig. 13

Fig. 14

Fig. 15

## INTERNATIONAL SEARCH REPORT

| International application No. |
|---|
| **PCT/JP2023/044585** |

### A. CLASSIFICATION OF SUBJECT MATTER

*A61L 27/06*(2006.01)i; *B21C 1/00*(2006.01)i; *C22C 14/00*(2006.01)i; *C22F 1/00*(2006.01)i; *C22F 1/18*(2006.01)i
FI: A61L27/06; B21C1/00 N; C22C14/00 Z; C22F1/00 604; C22F1/00 606; C22F1/00 624; C22F1/00 630A; C22F1/00 630C; C22F1/00 630K; C22F1/00 631A; C22F1/00 675; C22F1/00 685A; C22F1/00 694B; C22F1/00 694Z; C22F1/18 H

According to International Patent Classification (IPC) or to both national classification and IPC

### B. FIELDS SEARCHED

Minimum documentation searched (classification system followed by classification symbols)

A61L27/06; B21C1/00; C22C14/00; C22F1/00; C22F1/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2014/038487 A1 (MIURA, Hiromi) 13 March 2014 (2014-03-13) | 1-25 |
| | claims, paragraphs [0027]-[0058], examples | |
| Y | | 2, 6, 16, 21 |
| X | JP 2016-505387 A (INSTYTUT WYSOKICH CISNIEN POLSKIEJ AKADEMII NAUK) 25 February 2016 (2016-02-25) | 1-25 |
| | claims, paragraphs [0001]-[0004], examples | |
| Y | | 2, 6, 16, 21 |
| X | JP 2021-508764 A (SICHUAN UNIVERSITY) 11 March 2021 (2021-03-11) | 1-25 |
| | claims, paragraphs [0001]-[0008], examples | |
| Y | | 2, 6, 16, 21 |
| X | JP 2021-102225 A (UNIV TOYOHASHI TECHNOLOGY) 15 July 2021 (2021-07-15) | 1-25 |
| | claims, paragraphs [0002]-[0020], [0025]-[0032], examples | |
| Y | | 2, 6, 16, 21 |

[✓] Further documents are listed in the continuation of Box C.    [✓] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **01 February 2024** | **13 February 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2023/044585**

### C. DOCUMENTS CONSIDERED TO BE RELEVANT

| Category* | Citation of document, with indication, where appropriate, of the relevant passages | Relevant to claim No. |
|---|---|---|
| Y | JP 2020-121331 A (NIPPON STEEL CORPORATION) 13 August 2020 (2020-08-13) paragraph [0101] | 2, 6, 16, 21 |
| Y | JP 2019-510548 A (41 MEDICAL AG) 18 April 2019 (2019-04-18) paragraph [0065] | 2, 6, 16, 21 |
| P, X | WO 2022/259731 A1 (MARUEMU WORKS CO., LTD.) 15 December 2022 (2022-12-15) claims, examples | 1-25 |

Form PCT/ISA/210 (second sheet) (July 2022)

INTERNATIONAL SEARCH REPORT
Information on patent family members

International application No.

**PCT/JP2023/044585**

| Patent document cited in search report | | | Publication date (day/month/year) | Patent family member(s) | Publication date (day/month/year) |
|---|---|---|---|---|---|
| WO | 2014/038487 | A1 | 13 March 2014 | (Family: none) | |
| JP | 2016-505387 | A | 25 February 2016 | US 2015/0336147 A1 claims, paragraphs [0001]-[0004], examples WO 2014/092590 A1 EP 2931448 A1 | |
| JP | 2021-508764 | A | 11 March 2021 | WO 2019/100809 A1 claims, p. 1, line 2 to p. 2, line 9, examples CN 107881447 A | |
| JP | 2021-102225 | A | 15 July 2021 | (Family: none) | |
| JP | 2020-121331 | A | 13 August 2020 | (Family: none) | |
| JP | 2019-510548 | A | 18 April 2019 | US 2019/0029741 A1 paragraph [0085] WO 2017/139903 A1 EP 3416575 A1 | |
| WO | 2022/259731 | A1 | 15 December 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H7124242 A
- JP H9135852 A
- JP 5536789 B
- JP 6737686 B
- JP 2016506387 A
- JP 2021508764 A

**Non-patent literature cited in the description**

- **HIROAKI MATSUMOTO**. New type of ultrafine-grained microstructural formation technique of Pure-Ti and its development for practical application. *Heisei 26 Research Results Report (Public Interest Incorporated Foundation, The Kyoto Technoscience Center)*
- **KENZO KATO** ; **KINZOKU SOSEI KAKOUGAKU**. Metal Plastic Processing. Maruzen Co., Ltd., 1979, 208-212
- **ANTON POMP**. Steel Wire. Wire industry Ltd, 1954
- **WASEDA** ; **MATSUBARA** ; **SHINODA**. *Basics of Exercise X-ray Structural Analysis (Uchida Rokaku-ho)*, 2008, 103-108